# EUROPÄISCHE PATENTSCHRIFT

(11) **EP 2 667 834 B1**
(45) Veröffentlichungstag und Bekanntmachung des Hinweises auf die Patenterteilung: **16.07.2014**
(21) Anmeldenummer: 12700067.7
(22) Anmeldetag: 04.01.2012
(51) Int. Cl.: A61F 13/56, A61F 13/475

(54) **DAMEN- ODER INKONTINENZBINDE**
SANITARY OR INCONTINENCE PAD
SERVIETTE HYGIÉNIQUE OU POUR INCONTINENCE

(30) Priorität: 25.01.2011 DE 102011010269
(43) Veröffentlichungstag der Anmeldung: 04.12.2013
(73) Patentinhaber: Paul Hartmann AG, 89522 Heidenheim (DE)
(72) Erfinder: MALOWANIEC, Krzysztof, D., 89522 Heidenheim (DE)
(74) Vertreter: DREISS Patentanwälte PartG mbB
(86) Internationale Anmeldenummer: PCT/EP2012/050101
(87) Internationale Veröffentlichungsnummer: WO 2012/100970

(56) Entgegenhaltungen:
- EP-A1- 1 917 939
- EP-A1- 2 184 045
- EP-A2- 1 166 735
- WO-A1-96/38110
- US-A1- 2004 243 087

## Beschreibung

Die Erfindung betrifft eine Damen- oder Inkontinenzbinde mit um den Steg eines Unterbekleidungsstücks herum faltbaren Flügeln, wobei die Binde eine Längsrichtung und eine Querrichtung sowie einen in der Längsrichtung vorderen Endbereich und einen in der Längsrichtung hinteren Endbereich aufweist, mit einem mit seiner längeren Abmessung in der Längsrichtung erstreckten und einen Absorptionskörper für Körperflüssigkeiten aufnehmenden Bindenhauptteil und mit zwei Flügeln auf jeder Längsseite des Bindenhauptteils, die ausgehend von dem Bindenhauptteil in Querrichtung vorstehen, so dass sie ein vorderes und ein hinteres Flügelpaar bilden, wobei die Flügel eines Flügelpaars über Haftzonen miteinander oder mit einem Unterbekleidungsstück lösbar verbindbar sind und jeweils von einer vorderseitigen und von einer rückseitigen Flanke begrenzt werden, und mit beidseits entlang von Längsseitenrandbereichen des Bindenhauptteils erstreckten und befestigten Barriereelementen, die jeweils an ihrem distalen Längsrand ein gespanntes Elastifizierungsmittel aufweisen, welches bestrebt ist, das betreffende Barriereelement emporzuheben, wobei das jeweilige Barriereelement in einem vorderen und einem hinteren Längsabschnitt in eben gefalteter Konfiguration flächenhaft gegen eine Oberseite des Bindenhauptteils fixiert ist, so dass es sich in diesen Längsabschnitten nicht erheben kann.

Eine derartige Damen- oder Inkontinenzbinde mit zwei Flügeln auf jeder Seite ist beispielsweise vorbekannt aus WO 96/38110 A oder aus US 7,056,311 B2. Das primäre Ziel von sogenannten Flügeln bei Damen- oder Inkontinenzbinden ist es, die Binde am Unterbekleidungsstück lösbar zu fixieren und so in einer korrekten Position zu halten. Dies wird dadurch erreicht, dass die Flügel über Haftzonen an sich beliebiger Art, also insbesondere durch eine Haftkleberbeschichtung oder mechanische haken- bzw. schlaufenartige Befestigungsmittel, miteinander oder mit dem Unterbekleidungsstück lösbar haftend zusammenwirken. Hierbei werden die Flügel, vorzugsweise zumindest eines Flügelpaars, seitlich um die Längsränder des Stegs eines Unterbekleidungsstücks gefaltet und miteinander und/oder mit dem Unterbekleidungsstück über die vorerwähnten Haftzonen lösbar fixiert. Hierfür ist häufig auch ein Flügelpaar hinreichend. Wenn die Damen- oder Inkontinenzvorlage zusätzlich mit elastifizierten seitlichen Barriereelementen, die häufig auch als "Cuffs" bezeichnet werden, ausgestattet ist, so wirkt infolge der kontrahierenden Wirkung der Elastifizierungsmittel der Barriereelemente eine zusätzliche Kraft auf den Hygieneartikel, die nicht nur bestrebt ist, die Barriereelemente an deren distalem Längsrand emporzuheben, sondern auch insgesamt eine verformende Kraft auf den Hygieneartikel auszuüben. Insofern erweist es sich als vorteilhaft, wenn die bestimmungsgemäße Anordnung und lösbare Befestigung der Damen- oder Inkontinenzbinde durch zwei Flügelpaare, also durch jeweils zwei auf jeder Längsseite des Bindenhauptteils in Querrichtung vorstehende Flügel unterstützt wird. Die Flügel können - wie bereits erwähnt - je nach Form und Gestalt des Unterbekleidungsstücks um den Steg des Unterbekleidungsstücks herum geschlagen oder gewissermaßen von innen gegen eine körperzugewandte Oberseite des Unterbekleidungsstücks über ihre vorerwähnten Haftzonen lösbar festgelegt werden. In diesem Zusammenhang sei darauf hingewiesen, dass ein seitlich vorstehender Bereich einer hier in Rede stehenden Damen- oder Inkontinenzbinde dann als Flügel bezeichnet wird, wenn er eine Haftzone aufweist oder zum Herumschlagen um den Steg eines Unterbekleidungsstücks in eine lösbar haftende Konfiguration mit dem entsprechenden Flügelbereich auf der gegenüberliegenden Seite der Binde oder mit dem Unterbekleidungsstück ausgebildet ist.

Es wurde seither davon ausgegangen, dass sich das Vorhandensein von Flügeln und insbesondere das Herumschlagen der Flügel um den Steg eines Unterbekleidungsstücks gerade im Zusammenwirken mit vorzugsweise elastifizierten seitlichen Barriereelementen ("Cuffs") als vorteilhaft erweist, weil Flügel, insbesondere beim Herumfalten, eine Querzugkraft auf die seitlichen Barriereelemente ausüben und somit das Aufstehen oder Emporheben der Barriereelemente unterstützen oder propagieren und damit den Seitenauslaufschutz des Hygieneartikels verbessern.

Der vorliegenden Erfindung liegt die Aufgabe zu Grunde, eine Damen- oder Inkontinenzbinde der eingangs beschriebenen Art so weiter zu bilden, dass eine bestimmungsgemäße Positionierung der Binde und ihrer für die Flüssigkeitsabsorption wesentlichen Bestandteile bei der Benutzung weiter optimiert wird.

Diese Aufgabe wird bei einer Damen- oder Inkontinenzbinde der genannten Art erfindungsgemäß dadurch gelöst, dass sich der flächenhaft fixierte vordere und hintere Längsabschnitt des jeweiligen Barriereelements ausgehend von dem vorderen bzw. hinteren Endbereich in Längsrichtung wenigstens bis zu einem Punkt erstreckt, an dem der benachbarte Flügel die Hälfte seiner Fläche erreicht hat. Unter benachbartem Flügel wird dabei der dieser jeweiligen Längsseite zugeordnete sich in Querrichtung nach außen erstreckende Flügel verstanden.

Mit der vorliegenden Erfindung wurde nun festgestellt, dass die Flügel durchaus auch einen das bestimmungsgemäße Funktionieren des Cuff- oder Barriereelemente-Systems störenden oder gar verhindernden Einfluss haben können, in dem sie nämlich die Barriereelemente zu stark in Querrichtung nach außen ziehen, was dazu führen kann, dass die tatsächliche Erhebung in z-Richtung über eine flüssigkeitsaufnehmende Oberseite der Binde reduziert ist oder die Barriereelemente gar in unerwünschter Weise nach außen umgeklappt werden, der distale Längsrand, also in Querrichtung nach außerhalb einer sogenannten Cuff-Sockellinie der Barriereelemente gezogen wird. Beides wirkt sich aber negativ auf den Seitenauslaufschutz aus und erzeugt zusätzliche Kräfte innerhalb der Barriereelemente, die von der Konzeption des Hygieneartikels her unerwünscht oder jedenfalls nicht intendiert sind und sich in aller Regel negativ auf das Betriebsverhalten des Hygieneartikels auswirken. Mit der vorliegenden Erfindung wurde weiter erkannt, dass sich ein derartiger Einfluss der Flügel dadurch verhindern lässt, dass die jeweiligen Barriereelemente im Bereich der flächenhaften Erstreckung des hinteren und des vorderen Flügelpaars über einen wesentlichen Teil des Flügelpaars dauerhaft flächenhaft fixiert sind, also insbesondere in eine Ebene gefaltet und in dieser eben gefalteten Konfiguration gegen den Bindenhauptteil dauerhaft fixiert sind, sodass sie sich gerade nicht erheben können, sondern einen dauerhaft fixierten Streifen ergeben. In dem Bereich in Längsrichtung zwischen den Flügeln sind die Barriereelemente oder Cuffs dann so vorgesehen, dass sie sich unter der Wirkung ihres elastifizierten distalen Längsrands bestimmungsgemäß erheben können. In diesem Bereich wirkt sich dann auch das Umfalten des vorderen und des hinteren Flügelpaars nicht im vorausgehend geschilderten negativen Sinn aus, da dort keine Kraftkomponente auf das Barriereelemente-System übertragen wird. Es wurde erfindungsgemäß festgestellt, dass - wie mit Anspruch 1 der vorliegenden Anmeldung beansprucht - bei einer Mindesterstreckung des flächenhaft fixierten Längsabschnitts des jeweiligen Barriere-Elements in Längsrichtung von den Endbereichen in Richtung auf eine Mittelquerlinie oder ein Zentrum der Damen- oder Inkontinenzbinde wenigstens bis zu einem Punkt in Längsrichtung, an dem der betreffende Flügel die Hälfte (50%) seiner Fläche erreicht hat, zufriedenstellende Betriebseigenschaften des Hygieneartikels erreicht werden. Es erweist sich jedoch als vorteilhaft, wenn sich der flächenhaft fixierte vordere und hintere Längsabschnitt des jeweiligen Barriere-Elements ausgehend von dem vorderen bzw. hinteren Endbereich in Längsrichtung wenigstens bis zu einem Punkt erstreckt, an dem der benachbarte Flügel 60%, insbesondere 70% seiner Fläche erreicht hat.

Unter Zugrundelegung zweckmäßiger Abstände und/oder Gestaltungen des vorderen und hinteren Flügelpaars erweist es sich als vorteilhaft, wenn sich der flächenhaft fixierte vordere und/oder hintere Längsabschnitt höchstens oder weitestens bis zu einem Punkt erstreckt, an dem gewissermaßen 100 % der Fläche des betreffenden Flügels erreicht sind. Der flächenhaft fixierte Längsabschnitt des betreffenden Barriere-Elements würde sich dann in Längsrichtung gewissermaßen bis zum Ende des Flügels erstrecken, wo auch 100 % der Flügelfläche erreicht sind. Es erweist sich als vorteilhaft, wenn der betreffende fixierte Längsabschnitt sich in Längsrichtung höchstens bis zu einem Punkt erstreckt, an dem 90 %, insbesondere 80 % der Fläche des betreffenden Flügels erreicht sind.

Zur Abgrenzung der Flügel vom Bindenhauptteil wird jeweils beidseitig an die zwischen dem vorderen und dem hinteren Flügelpaar schmalste Stelle der Binde, d.h. an dem Punkt an dem die Kontur in Querrichtung die geringste Erstreckung aufweist, eine zur Längsrichtung der Binde parallele Gerade oder Tangente angelegt. Diejenigen Bereiche der Binde in Querrichtung außerhalb dieser Geraden oder Tangenten werden den Flügeln zugeordnet bzw. für die Bestimmung der Fläche oder der Aufsichtsfläche der Flügel herangezogen. Diese angelegten parallelen Geraden oder Tangenten tragen zur Bestimmung der Fläche oder Aufsichtsfläche der Flügel im Sinne der vorliegenden Erfindung bei, wobei die schmalste Stelle zwischen dem vorderen und dem hinteren Flügelpaar im Zweifel die vorderen und hinteren Flügel voneinander trennt oder unterscheidet. - Vorzugsweise weist eine erfindungsgemäße Binde auf jeder Seite genau zwei Flügel, also ein hinteres und ein vorderes Flügelpaar auf.

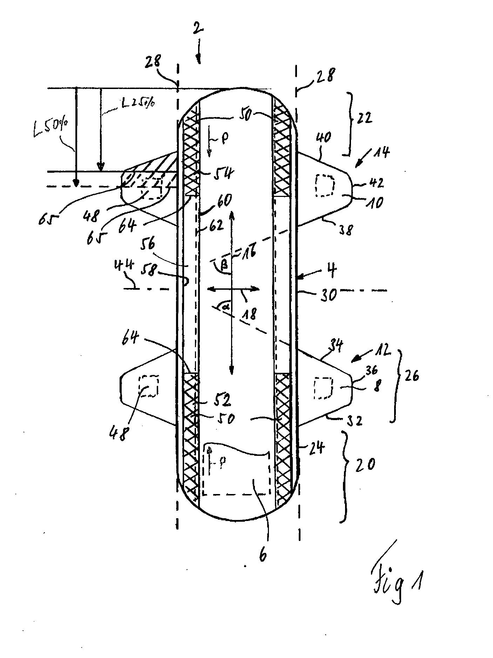

Hinsichtlich der Erstreckung des flächenhaft fixierten, also nicht emporhebbaren Längsabschnitts des jeweiligen Barriereelements wird jeweils von vom bzw. hinten ausgegangen und dann in Richtung auf die Quermittellinie oder das Zentrum des Hygieneartikels vorangeschritten. Es sind dies die in den Figuren kreuzschraffiert dargestellten Bereiche der jeweiligen Barriereelemente. In dieser Richtung wird auch die zunehmende Fläche der Flügel betrachtet, also ebenfalls von vom bzw. hinten in Richtung der in Figur 1 eingezeichneten Pfeile P. Eine Längserstreckung L 25% gibt daher einen Punkt oder eine Stelle in Längsrichtung an, bei der der hintere Flügel etwa 25% seiner Fläche erreicht hat. Eine Längserstreckung L 50% in Figur 1 gibt einen Punkt oder eine Stelle an, an der der betreffende Flügel ca. 50% seiner flächenhaften Erstreckung erreicht hat. Die Fläche wird dabei durch die vorerwähnte zur Längsrichtung parallele Gerade oder Tangente an die schmalste Stelle und durch eine parallel zur Querrichtung der Binde verlaufende Linie begrenzt, so wie dies aus der Figur 1 ersichtlich ist (schraffierter Bereich). Man erkennt, dass der kreuzschraffiert dargestellte flächenhaft fixierte Längsabschnitt des angrenzenden Barriere-Elements sich in Längsrichtung in Richtung des Pfeils P über die Längserstreckung L 50% hinaus erstreckt, so wie dies gemäß Anspruch 1 der vorliegenden Erfindung gefordert wird.

Im fixierten vorderen und hinteren Längsabschnitt des Barrieremittels ist das Barrieremittel insbesondere durch Kleber, durch thermische und/oder Ultraschallschweißstellen oder Kombinationen an die Oberfläche des Bindenhauptteils dauerhaft festgelegt.

Es erweist sich in weiterer Ausbildung der Erfindung als vorteilhaft, wenn das jeweilige Barriere-Element im Bereich zwischen den Flügeln nur entlang einer proximalen Linie, der sogenannten Cuff-Sockellinie, mit dem Bindenhauptteil fixiert ist, sodass es sich mit seinem distalen elastifizierten Längsrand emporheben kann. Solchenfalls steht gewissermaßen die gesamte Breite des Barriere-Elements, also der Bereich zwischen der Cuff-Sockellinie und dem distalen elastifizierten Längsrand des Barriere-Elements als Seitenauslaufschutzbarriere zur Verfügung, in dem sich das Barriere-Element unter der Wirkung seines elastifizierten distalen Längsrands von der Oberseite des Bindenhauptteils abhebt. Es werden hierbei gut funktionierende Taschen zwischen dem Barriere-Element und der Oberseite des Bindenhauptteils gebildet.

Die Fixierung des Barriere-Elements entlang der Cuff-Sockelline erfolgt vorzugsweise mittels Schweißverfahren, wie thermisches Schweißen oder Ultraschallschweißen und den damit erzeugten Schweißstellen oder mittels adhäsiver Mittel oder Kombinationen der genannten Verfahren oder Mittel.

Als Elastifizierungsmittel für die Elastifizierung des distalen Längsrandes des Barriere-Elements werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden. Die Elastifizierungsmittel haben vorzugsweise eine Stärke von 300-1500 dtex, insbesondere von 500 - 1200 dtex, weiter insbesondere von 500 - 900 dtex. Alternativ können auch Schaumstoffbänder eingesetzt sein.

Die Elastifizierungsmittel werden vorzugsweise unter einer Vorspannung von 1,5 - 6,0, insbesondere von 2,5 - 4,5 an den Barriere-Elementen fixiert.

Weiter erweist sich als vorteilhaft, wenn die Flügel des vorderen Flügelpaars mit wenigstens 90% ihrer Aufsichtsfläche innerhalb eines Längsabschnittsbereichs der Binde angeordnet sind, der höchstens ein Drittel (1/3), insbesondere höchstens ein Viertel (1/4) ihrer Gesamtlängserstreckung erfasst. Mit anderen Worten ausgedrückt bedeutet dies, dass ein Großteil der Flügel des vorderen Flügelpaars auf einen höchstens ein Drittel, höchstens ein Viertel der Bindenlänge ausmachenden Längsabschnittsbereich konzentriert ist.

Weiter erweist sich als vorteilhaft, wenn der vordere Endbereich in Längsrichtung höchstens ein Drittel (1/3), insbesondere höchstens zwei Siebtel (2/7), insbesondere wenigstens ein Siebtel (1/7), insbesondere wenigstens ein Sechstel (1/6), insbesondere wenigstens ein Fünftel (1/5) der Gesamtlängserstreckung der Binde erfasst und die Flügel des vorderen Flügelpaars mit wenigstens 90% ihrer Aufsichtsfläche in Längsrichtung außerhalb des vorderen Endbereichs angeordnet sind.

Es erweist sich als zweckmäßig und vorteilhaft, wenn die Erstreckung der Flügel des vorderen und/oder hinteren Flügelpaars in Querrichtung über die schmalste Stelle der Binde zwischen dem vorderen und dem hinteren Flügelpaar hinaus 20 - 70 mm, insbesondere 20 - 50 mm, insbesondere 25 -45 mm beträgt. Hierbei wird zur Abmessung die Erstreckung in Querrichtung zwischen der imaginär an die schmalste Stelle angelegte Tangente/parallelen Geraden und dem Punkt der maximalen Erstreckung des Flügels außerhalb dieser imaginären Linie herangezogen.

Weiter erweist es sich als vorteilhaft, wenn die Aufsichtsfläche jedes Flügels des hinteren Flügelpaars und/oder die Aufsichtsfläche jedes Flügels des vorderen Flügelpaars jeweils einen Anteil von vorzugsweise 3 - 10 %, weiter vorzugsweise 3 - 8 %, weiter vorzugsweise 3 - 7 %, weiter vorzugsweise von 4 - 6 % bezogen auf die Gesamtfläche der Damen- oder Inkontinenzbinde einnimmt. Der im Vergleich zur Gesamtfläche in diesem Fall geringe flächenhafte Anteil des hinteren Flügels ermöglicht eine insbesondere zur Anbindung des Flügels an die Innenseite des Unterbekleidungsstücks ausreichende Erstreckung unter Vermeidung von versteifenden Widerständen durch Materialanhäufung in Längsrichtung der Binde.

Weiter erweist es sich als vorteilhaft, wenn die Aufsichtsfläche jedes Flügels des vorderen Flügelpaars jeweils 10-17cm², weiter vorzugsweise 11-16cm², weiter vorzugsweise 12 -15cm², weiter vorzugsweise 13-14cm ² und/oder die jedes Flügels des hinteren Flügelpaars jeweils 10-17cm², weiter vorzugsweise 11-16cm², weiter vorzugsweise 12-15cm², weiter vorzugsweise 13-14cm² beträgt.

Die Gesamtlängserstreckung der Damen- oder Inkontinenzbinde entlang der Längsrichtung beträgt bei einer bevorzugten Ausführungsform 150 - 400 mm, insbesondere 150 - 350 mm, insbesondere 200 - 350 mm, insbesondere 200 - 300 mm, insbesondere 250 - 300 mm, insbesondere 275 - 290 mm.

Um eine möglichst kompakte Ausbildung und Anordnung der Flügel in einem begrenzten eher kurzen Längsabschnittsbereich der Binde zu realisieren, jedoch dennoch eine möglichst große Fläche der Flügel zur Anbindung an das Unterbekleidungsstück zu haben, erweist es sich als vorteilhaft, wenn die vorderseitigen und die rückseitigen Flanken der Flügel des vorderen und/oder hinteren Flügelpaars mit dem überwiegenden Teil ihrer Bahn in einem Winkel (α, β) > 50°, insbesondere > 60°, insbesondere > 65° zur Längsrichtung verlaufen, wobei der Winkel weiter insbesondere 60 - 85°, insbesondere 60 - 75° beträgt. Vorliegend ist dabei der kleinere der beiden Schnittwinkel zwischen der Flankenrichtung und der Längsrichtung gemeint. Bei einer derartigen Ausbildung werden sich die Flügel in Querrichtung nach außen verjüngen, wobei ihr Verlauf durchaus steil ist, was große Flügelflächen bei vergleichsweise moderater Flügelerstreckung in Längsrichtung gestattet.

Im Hinblick auf die Konturierung der Flügel könnten beispielsweise die jeweiligen vorderseitigen Flanken verrundet unmittelbar in die rückseitigen Flanken übergehen. Es wäre aber auch denkbar, dass zwischen der vorderseitigen und der rückseitigen Flanke eines jeweiligen Flügels ein weiterer gegenüber diesen insbesondere kürzerer Flankenabschnitt vorgesehen ist, der weiter insbesondere im Wesentlichen in Längsrichtung verläuft und in den die Flanken vorzugsweise verrundet übergehen.

Zwar wäre es denkbar, dass im Übergang zwischen einem Längsrand des Bindenhauptteils und einer vorderseitigen oder rückseitigen Flanke der Flügel ein unstetiger Konturverlauf vorgesehen ist, wodurch im Übrigen die Ausbildung einer Biege- oder Falzlinie vorgegeben werden kann. Indessen kann es sich auch als vorteilhaft erweisen, wenn die Außenkontur der Binde verrundet ausgebildet ist. In Weiterführung dieses Gedankens erweist es sich als vorteilhaft, wenn ein Krümmungsradius im Übergang zwischen einem im Wesentlichen in der Längsrichtung erstreckten Längsrand des Bindenhauptteils und der vorderseitigen Flanke und/oder zwischen der vorderseitigen Flanke und dem mittleren kürzeren Flankenabschnitt und/oder zwischen dem mittleren kürzeren Flankenabschnitt und der rückseitigen Flanke und/oder zwischen der rückseitigen Flanke und dem im Wesentlichen in der Längsrichtung erstreckten Längsrand des Bindenhauptteils eines jeweiligen Flügels 5 -12 mm, insbesondere 5 - 10 mm, insbesondere 6 - 9 mm beträgt.

Nach einem weiteren Erfindungsgedanken, der sich auch in optischer Hinsicht vorteilhaft auswirken kann, wird vorgeschlagen, die Flügelkonturen so auszubilden, dass die rückseitigen Flanken des vorderen Flügelpaars und die vorderseitigen Flanken des hinteren Flügelpaars mit dem überwiegenden Teil ihrer Bahn gerade verlaufen.

Weiter kann es sich als vorteilhaft erweisen, wenn die vorderseitigen Flanken des vorderen Flügelpaars und/oder die rückseitigen Flanken des hinteren Flügelpaars bogenförmig gekrümmt, vorzugsweise unter Ausbildung einer nach außen gewölbten Kontur verlaufen, wobei solchenfalls vorzugsweise nur eine sehr leichte bogenförmige Krümmung mit einem Krümmungsradius in der Größenordnung von 100 mm und mehr, insbesondere mit einem Krümmungsradius von 120 - 180 mm, weiter insbesondere von 140 - 160 mm, weiter insbesondere von 150 - 155 mm vorgesehen ist.

Weiter kann es sich als vorteilhaft erweisen, wenn die vorderseitigen Flanken des vorderen Flügelpaares und die rückseitigen Flanken des hinteren Flügelpaars gekrümmt verlaufen.

Die Krümmung der vorderseitigen Flanken des vorderen Flügelpaares und/oder der rückseitigen Flanken des hinteren Flügelpaars trägt in vorteilhafter Weise zur ergonomischen Passform der Inkontinenz- oder Damenbinde im Gebrauchszustand bei. Im Gebrauchszustand der Faltung der Flügel des vorderen Flügelpaares um den Schrittsteg herum auf die Außenseite des Unterbekleidungsstücks und der Anbindung der Flügel des hinteren Flügelpaares gegen die Innenseite des Unterbekleidungsstücks unterstützt die Krümmung der Flanken der Flügel die flexible Anpassung an die dreidimensionale Gestalt der Binde.

Obschon die vorderseitigen Flanken des vorderen Flügelpaars und/oder die rückseitigen Flanken des hinteren Flügelpaars nicht gerade, sondern vorzugsweise leicht bogenförmig gekrümmt verlaufen können, so lässt sich hier ebenfalls eine Tangente an den Konturverlauf anlegen, die mit der Längsrichtung einen Winkel > 50°, insbesondere > 60°, insbesondere > 65° zur Längsrichtung einschließt, wobei der Winkel weiter insbesondere 60 - 85°, insbesondere 60 - 75° beträgt.

Bei einer besonders vorteilhaften Ausführungsform der Damen- oder Inkontinenzbinde sind die vorderseitigen Flanken des vorderen Flügelpaars und die rückseitigen Flanken des hinteren Flügelpaars einander entsprechend konturiert, so dass sie bei Faltung der Binde um eine in Querrichtung (also senkrecht zur Längsachse) verlaufende Achse in Deckung aufeinander bringbar sind.

Weiter erweist sich als vorteilhaft, wenn die Flügel des hinteren Flügelpaars mit wenigstens 90% ihrer Aufsichtsfläche innerhalb eines Längsabschnittsbereichs der Binde angeordnet sind, der höchstens ein Drittel (1/3), insbesondere höchstens ein Viertel (1/4) ihrer Gesamtlängserstreckung erfasst. Mit anderen Worten ausgedrückt bedeutet dies, dass ein Großteil der Flügel des hinteren Flügelpaars auf einen höchstens ein Drittel, höchstens ein Viertel der Bindenlänge ausmachenden Längsabschnittsbereich konzentriert ist.

Weiter erweist sich als vorteilhaft, wenn der hintere Endbereich in Längsrichtung höchstens ein Drittel (1/3), insbesondere höchstens zwei Siebtel (2/7), insbesondere wenigstens ein Siebtel (1/7), insbesondere wenigstens ein Sechstel (1/6), insbesondere wenigstens ein Fünftel (1/5) der Gesamtlängserstreckung der Binde erfasst und die Flügel des hinteren Flügelpaars mit wenigstens 90% ihrer Aufsichtsfläche in Längsrichtung außerhalb des hinteren Endbereichs angeordnet sind.

Alternativ kann es sich als vorteilhaft erweisen, wenn die Flügel des hinteren Flügelpaars zugleich den hinteren Abschluss der Damen- oder Inkontinenzbinde bilden.

Insbesondere vorteilhaft sind wenigstens 90% der Aufsichtsfläche des hinteren Flügelpaares innerhalb des hinteren Endbereichs angeordnet, wobei der hintere Endbereich höchstens ein Viertel (1/4), insbesondere höchstens ein Fünftel (1/5), insbesondere höchstens ein Sechstel (1/6) der Gesamtlängserstreckung der Binde erfasst.

Hierbei erweist es sich als vorteilhaft, wenn die rückseitigen Flanken des hinteren Flügelpaars in ergonomischer Fortsetzung ihrer Bahn nach innen aufeinander zustreben und so den hinteren Endbereich des Bindenhauptteils begrenzen. Die Flügel des hinteren Flügelpaars gehen also in gewisser Weise ergonomisch einstückig in den Bindenhauptteil über, so dass der Bindenhauptteil in Längsrichtung keinen weitergehenden Fortsatz in rückwärtiger Richtung der Binde aufweist.

Die Flügel der Damen- oder Inkontinenzbinde weisen zur Festlegung an das Unterbekleidungsstück vorteilhafterweise Haftzonen auf. Die Haftzonen sind dabei auf der Unterseite der Flügel vorgesehen. Es erweist sich weiter als vorteilhaft, wenn die Fläche der jeweiligen Haftzone pro Flügel an die Aufsichtsfläche des Flügels angepasst ist. Die Haftzone weist je Flügel vorzugsweise eine Fläche von 20 - 50 %, weiter vorzugsweise von 20 - 40 % bezogen auf die Aufsichtsfläche des Flügels auf. Dabei ist die Haftzone vorteilhafterweise mittig zwischen der vorderseitigen und der rückseitigen Flanke des jeweiligen Flügels angeordnet.

Auch bei der Befestigung der Binde mittels der Haftzonen ist die insbesondere bei den Flügeln des hinteren Flügelpaares kurze Erstreckung in Längsrichtung vorteilhaft, da an sich große flächenhafte Erstreckungen der Flügel zur Vermeidung von Materialverfaltungen beim Gebrauch eines größeren Anteils an Haftzonen bedürfen, was sich wiederum nachteilig infolge ihrer versteifenden Wirkung in diesen Bereichen zeigen würde.

Weiter vorteilhaft ist der Bindenhauptteil der Damen- oder Inkontinenzbinde auf der Unterseite, also auf der dem im Gebrauch vorhandenen Unterbekleidungsstück zugewandten Seite mit weiteren Haftzonen versehen. Diese weiteren Haftzonen können an sich beliebig, insbesondere in Form von mehreren in Längsrichtung des Bindenhauptteils erstreckten Streifen angeordnet sein.

Die Haftzonen werden vorzugsweise durch eine Haftkleberbeschichtung, durch mechanische Haken und/oder Klettschlaufenelemente und/oder durch Kombinationen davon bereitgestellt.

Herstellerseitig können die Damen- oder Inkontinenzbinden in eben ausgebreitetem Zustand, insbesondere in Stapelform, an den Letztverbraucher abgegeben werden. Es wäre indessen auch denkbar, dass die Binde um eine oder mehrere in Querrichtung verlaufende Faltachsen auf sich selbst gefaltet ist, so dass ihre Längserstreckung vor dem Gebrauch hierdurch erheblich reduziert ist. Unabhängig von einer solchen Faltung der Binde um eine oder mehrere Querachsen können die vorderen und/oder die hinteren Flügel auf die Oberseite oder die Unterseite des Bindenhauptteils herstellerseitig eingefaltet sein, um die Quererstreckung des Produkts bei der Lagerung, im Handel und bei der Abgabe an den Endverbraucher zu reduzieren. Eine hierfür vorgesehene Längsfaltlinie verläuft dabei typischer Weise in Längsrichtung der Binde und kann auch Randbereiche des Bindenhauptteils außerhalb der eigentlichen Flügel erfassen. Jedoch verläuft eine solche herstellerseitig vorgenommene Längsfaltlinie vorzugsweise außerhalb des Absorptionskörpers der Binde.

Es kann sich als vorteilhaft erweisen, wenn die Flügel des vorderen und/oder des hinteren Flügelpaars herstellerseitig auf eine körperzugewandte Oberseite der Binde geschlagen sind und möglicherweise einander dabei überlappen und wenn eine dann nach oben gewandte Haftbeschichtung bei den jeweiligen Flügeln je Flügelpaar von einem einzigen ablösbaren flächenhaften Schutzelement, insbesondere auf Papier- oder Folienbasis, insbesondere mit Antihaftmitteln beschichtet, überfangen ist, welches beide Flügel erfasst.

Die Flügel des vorderen und hinteren Flügelpaares umfassen vorzugsweise oder bestehen vorzugsweise aus einem die Oberseite der Damen- oder Inkontinenzbinde bildenden Deckblatt-Material und aus einem die Unterseite der Damen- oder Inkontinenzbinde bildenden Backsheet-Material. Die Flügel sind vorzugsweise frei von zwischen Deckblatt- und Backsheet-Material angeordneten Absorptionsmaterialien.

Der Absorptionskörper erstreckt sich vorzugsweise innerhalb des Bindenhauptteils, und ist insbesondere rechteckförmig ausgebildet und in Längsrichtung der Binde erstreckt. Es ist auch denkbar, dass die Form des Absorptionskörpers in den Endbereichen dem Konturverlauf der Endbereiche des Bindenhauptteils angepasst ist.

Für sämtliche vorausgehend beschriebenen Merkmale wird Schutz in jeder beliebigen Kombination in Anspruch genommen. Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Damen- oder Inkontinenzbinde.

In der Zeichnung zeigt
Figur 1 eine Draufsicht auf eine Damen- oder Inkontinenzbinde in eben ausgefaltetem Zustand;
Figur 2 eine perspektivische schematisierte Ansicht einer ungefähr der Figur 1 entsprechenden Damen- oder Inkontinenzbinde mit angedeuteten auf die körperabgewandte Seite der Binde umgeschlagenen Flügeln;
Figur 3 eine Draufsicht auf eine weitere Ausführungsform einer erfindungsgemäßen Damen- oder Inkontinenzbinde in eben ausgefaltetem Zustand;
Figur 4 eine perspektivische schematisierte Ansicht einer ungefähr der Figur 3 entsprechenden Damen- oder Inkontinenzbinde, wobei nur die Flügel des vorderen Flügelpaars auf die körperabgewandte Seite der Binde umgeschlagen sind;
Figuren 5,6 schematische perspektivische Darstellungen in Längsrichtung betrachtet einer erfindungsgemäßen Damen- oder Inkontinenzbinde mit nicht umgeschlagenen bzw. umgeschlagenen Flügeln.

Figur 1 zeigt eine insgesamt mit dem Bezugszeichen 2 bezeichnete Damen- oder Inkontinenzbinde oder -vorlage mit einem Bindenhauptteil 4, der einen im beispielhaft dargestellten Fall rechteckförmigen Absorptionskörper 6 umfasst, und mit zwei Flügeln 8 und 10 auf jeder Seite. Die vorderen Flügel 8 bilden ein vorderes Flügelpaar 12 und die hinteren Flügel 10 bilden ein hinteres Flügelpaar 14.

Mit Bezugszeichen 16 ist eine Längsrichtung und mit Bezugszeichen 18 eine hierzu senkrechte Querrichtung der Damen- oder Inkontinenzbinde 2 bezeichnet. Ferner ist in Figur 1 angedeutet ein vorderer Endbereich 20 und ein hinterer Endbereich 22. Im vorderen Endbereich 20 sind keine Flügel vorhanden, sondern der Bindenhauptteil 4 begrenzt die Binde mit seinen im Wesentlichen in Längsrichtung verlaufenden Längsrändern 24. An diesen vorderen Endbereich 20 schließt sich ein Längsabschnittsbereich 26 an, der beispielhaft höchstens ein Viertel der Gesamtlängserstreckung der Damen- oder Inkontinenzbinde umfasst, und innerhalb dessen die Flügel 8 des vorderen Flügelpaars 12 mit ihrer im Wesentlichen gesamten flächenhaften Erstreckung angeordnet sind. In entsprechender Weise sind die Flügel 10 des hinteren Flügelpaars 14 ebenfalls mit dem Großteil ihrer flächenhaften Erstreckung in Längsrichtung außerhalb des hinteren Endbereichs 22 vorgesehen, was aber nicht zwingend sondern lediglich beispielhaft ist.

Zur Abgrenzung der Flügel 8, 10 vom Bindenhauptteil 4 ist in Figur 1 jeweils beidseitig eine parallele Gerade oder Tangente 28 an die schmalste Stelle 30 der Binde zwischen dem vorderen und dem hinteren Flügelpaar 12, 14 gelegt. Diejenigen Bereiche der Binde in Querrichtung 18 außerhalb dieser Geraden oder Tangenten 28 werden den Flügeln 8, 10 zugeordnet.

Die Flügel 8 des vorderseitigen Flügelpaars 12 werden von vorderseitigen Flanken 32 und rückseitigen Flanken 34 sowie von einem zwischen der vorderseitigen und der rückseitigen Flanke zu liegen kommenden kürzeren Flankenabschnitt 36 begrenzt, der im Wesentlichen in Längsrichtung 16 verläuft. Auch die Flügel 10 des hinteren Flügelpaars 14 werden von einer vorderseitigen Flanke 38 und einer rückseitigen Flanke 40 sowie von einem mittleren kürzeren Flankenabschnitt 42 begrenzt.

Die Flanken 32, 34 bzw. 38, 40 sind im beispielhaft dargestellten Fall im Wesentlichen gerade erstreckt, was aber nicht zwingend ist. Sie gehen verrundet in die jeweiligen mittleren kürzeren Flankenabschnitte 36 bzw. 42 über.

Die Flanken 32, 34 des vorderen Flügelpaars 12 und die Flanken 38, 40 des hinteren Flügelpaars 14 schneiden die Längsrichtung 16 in einem Winkel α bzw. β, der vorzugsweise > 50° beträgt und insbesondere zwischen 60° und 85° liegt, wobei auch dies rein beispielhaft ist. Auf diese Weise werden in der Längsrichtung 16 verhältnismäßig kurz erstreckte Flügel 8, 10 gebildet, die eine sehr gute Handhabbarkeit beim Befestigen an einem Unterbekleidungsstück aufweisen und auch einen hohen Tragkomfort vermitteln.

Die vorderseitigen Flanken 32 des vorderen Flügelpaars 12 und die rückseitigen Flanken 40 des hinteren Flügelpaars 14 sind einander entsprechend konturiert. Wenn man die Binde um eine in Querrichtung verlaufende Achse 44 auf sich selbst faltet, so koinzidiert der Verlauf der vorderseitigen Flanken 32 des vorderen Flügelpaars 12 und der rückseitigen Flanken 40 des hinteren Flügelpaars 14. Rein beispielhaft koinzidieren auch die einander zugewandten Flanken 34, 38.

Ferner sind mechanisch oder klebend haftend ausgebildete Haftzonen 48 bei den Flügeln dargestellt.

Weiter umfasst die Damen- oder Inkontinenzbinde 2 beidseits entlang von Längsseitenrandbereichen des Bindenhauptteils 4 erstreckte und dort auf noch näher zu beschreibende Weise befestigte Barriereelemente 50, die im beispielhaft dargestellten Fall über die gesamte Längserstreckung der Binde erstreckt sind. Sie sind in dem kreuzschraffiert dargestellten Längsabschnitt 52 vom und 54 hinten flächenhaft eben auf die Oberseite des Bindenhauptteils 4 aufgelegt und dort flächenhaft, das heißt über den gesamten kreuzschraffiert dargestellten Längsabschnitt 52 bzw. 54 dauerhaft fixiert, etwa durch Kleber, durch Prägung oder sonstige an sich übliche Fügeverfahren.

In dem Bereich 56 zwischen den Längsabschnitten 52 und 54 sind die beidseitigen Barriereelemente 50 nur entlang einer proximalen Linie 58, der sogenannten Cuff-Sockellinie an dem Bindenhauptteil 4 fixiert. Die beidseitigen Barriereelemente 50 sind an ihrem distalen Längsrand 60 durch wenigstens ein gespanntes Elastifizierungsmittel 62 elastifiziert, sodass sie sich in dem Bereich 56 in Folge der Spannung der Elastifizierungsmittel 62 von der Oberseite des Bindenhauptteils erheben können, um eine wirksame Seitenauslaufsperre zu bilden.

Man erkennt aus Figur 1, dass sich die kreuzschraffiert dargestellten flächenhaft fixierten Längsabschnitte 52, 54 der Barriereelemente 50 ausgehend von den Endbereichen 20, 22 in Richtung P auf die Quermittellinie 44 oder ein Zentrum der Binde bis zu einem Punkt oder einer Stelle 64 erstrecken, an dem der benachbarte Flügel 8, 10 wenigstens die Hälfte seiner Fläche erreicht hat. Derjenige Punkt in Längsrichtung oder diejenige Längserstreckung, an dem oder der der betreffende Flügel die Hälfte, also 50% seiner Fläche erreicht hat (in Richtung des Pfeils P), ist in Figur 1 mit L 50% bezeichnet. Ein zu 25% der Fläche gehöriger Punkt ist mit L 25% bezeichnet. Die Fläche wird dabei durch die vorerwähnte zur Längsrichtung 16 parallele Gerade oder Tangente 28 an die schmalste Stelle 30 und durch eine parallel zur Querrichtung 18 der Binde verlaufende Linie 65 begrenzt, so wie dies aus der Figur 1 ersichtlich ist (schraffierter Bereich).

Auf diese Weise ist erfindungsgemäß erreicht worden, dass die Flügel 8, 10 insbesondere beim Umfalten auf die körperabgewandte Seite der Binde 2 in geringerem Maße die bestimmungsgemäße Funktion der seitlichen Barriereelemente 50 im Betrieb beeinträchtigen.

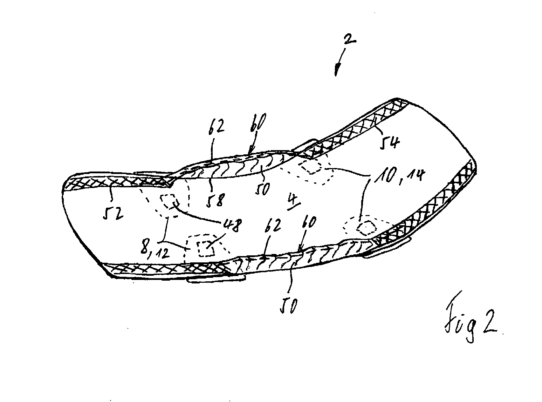

Figur 2 zeigt eine perspektivische Ansicht einer Damen- oder Inkontinenzbinde 2, bei der es sich insbesondere um die in Figur 1 dargestellte Damenbinde handeln kann, wobei die Flügel 8, 10 auf die körperabgewandte Seite der Binde geschlagen sind. Aufgrund der unter Spannung stehenden Elastifizierungselemente 62 am jeweiligen distalen Längsrand 60 der beidseitigen Barriereelemente 50 erkennt man, wie die Barriereelemente 50 in dem Bereich in Längsrichtung zwischen dem vorderen und hinteren Flügelpaar 12, 14 sich von der Oberseite des Bindenhauptteils 4 erheben können und so einen wirkungsvollen Seitenauslaufschutz bilden können. In dem betreffenden vorderen und hinteren Längsabschnitt 52 bzw. 54 der Barriereelemente 50 ist dies nicht möglich, da die Barriereelemente dort vollflächig mit der Oberfläche des Bindenhauptteils 4 verbunden sind.

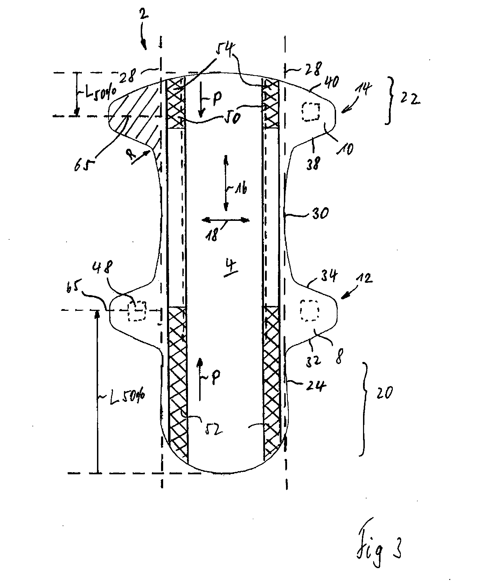

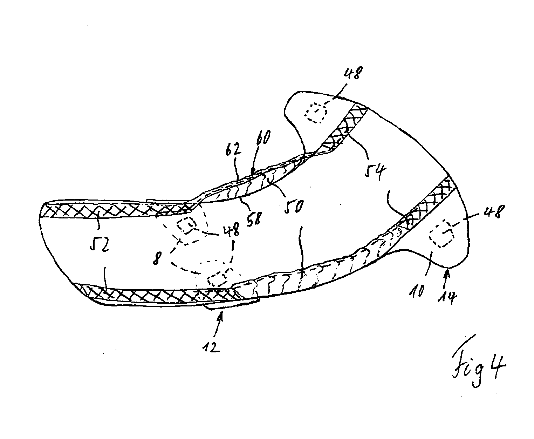

Die Figuren 3 und 4 zeigen eine Draufsicht und eine perspektivische Ansicht einer weiteren Ausführungsform einer erfindungsgemäßen Damen- oder Inkontinenzbinde oder Vorlage 2, wobei entsprechende Bezugszeichen verwendet wurden. Im Wesentlichen unterscheidet sich die Binde 2 gemäß Figuren 3, 4 von der in Figuren 1 und 2 dargestellten Binde dadurch, dass deren Flanken 32, 34 bzw. 38, 40 nicht exakt gerade verlaufen und auch der Übergang der Flanken zu seitlichen Längsrändern 24 des Bindenhauptteils 4 nicht exakt parallel zur Längsrichtung 16 verläuft. Außerdem ist das hintere Flügelpaar 14 im hinteren Endbereich 22 der Binde 2 vorgesehen, und zwar beispielhaft derart, dass die jeweilige rückseitige Flanke 40 der hinteren Flügel 10 zugleich den hinteren Querrand bildet und damit den hinteren Endbereich 22 der Binde begrenzt.

Zur Bestimmung der Fläche der Flügel 8, 10 wird wiederum an den schmalsten Bereich in Querrichtung 18 zwischen dem vorderen und dem hinteren Flügelpaar 12, 14 eine zur Längsrichtung 16 parallele Gerade oder Tangente 28 angelegt, um die Fläche der Flügel 8, 10 zu begrenzen. Diese Fläche ist durch die Schraffur des hinteren Flügels 10 in der Figur 3 links angegeben. Wiederum ist mit L 50% diejenige Stelle in Längsrichtung 16 ausgehend vom hinteren Endbereich 22 in Richtung des Pfeils P angegeben, bei der der betreffende Flügel 10 50% seiner Fläche erreicht hat, und zwar begrenzt durch die Gerade oder Tangente 28 und durch die zur Querrichtung 18 parallele Linie 65. Man erkennt, dass der betreffende Längsabschnitt 52, 54 der beidseitigen Barriereelemente 50, innerhalb dessen die Barriereelemente flächenhaft auf die Oberseite des Bindenhauptteils 4 dauerhaft festgelegt sind, sich über dieses Maß L 50% hinauserstrecken. Entsprechend ist bei dem vorderen Flügelpaar L 50% eingezeichnet.

Man kennt auch das die Flanken 32, 34 bzw. 38, 40 der vorderen und hinteren Flügel 8, 10 verrundet in die seitlichen Längsränder 24 des Bindenhauptteils übergehen. Folgt man dem Flankenverlauf, so erkennt man jeweils eine Stelle eines kleinsten Krümmungsradius R.

In der perspektivischen Darstellung nach Figur 4 sind die Flügel 8 des vorderen Flügelpaares 12 auf die körperabgewandte Seite der Binde umgeschlagen, und sie werden dort über die Haftzonen 48 lösbar an der körperabgewandten Seite des Stegs eines Unterbekleidungsstücks befestigt. Die Flügel 10 des hinteren Flügelpaars 14 sind indessen nicht umgefaltet. Dadurch, dass sie im hinteren Endbereich 22 der Binde 2 angeordnet sind lassen sie sich je nach der Geometrie des nicht dargestellten Unterbekleidungsstücks mit ihren Haftzonen 48 auch gegen die körperzugewandte Innenseite des Unterbekleidungsstücks haftend lösbar festlegen. Wiederum erkennt man, dass sich die Barriereelemente in dem Bereich in Längsrichtung 16 zwischen dem vorderen und hinteren Flügelpaar 12, 14 im wesentlichen unbeeinflusst durch den Betriebszustand der Flügel 8, 10 erheben können, und zwar hauptsächlich in Folge der in Längsrichtung 16 verlaufenden Spannung der Elastifizierungselemente 62 am distalen Längsrand 60 der Barriereelemente 50. In diesem Bereich sind die Barriereelemente 50 vorzugsweise nur entlang der proximalen Linie 58, der sogenannten Cuff-Sockellinie, mit dem Bindenhauptteil 4 verbunden. Es wäre indessen auch denkbar, dass die Barriereelemente 50 in Querrichtung 18 außerhalb der proximalen Linie 58 über einige Millimeter flächenhaft fixiert sind. In jedem Fall ist derjenige fixierte Bereich aber wesentlich schmaler als der über die gesamte Breite flächenhaft fixierte Längsabschnitt 52 bzw. 54.

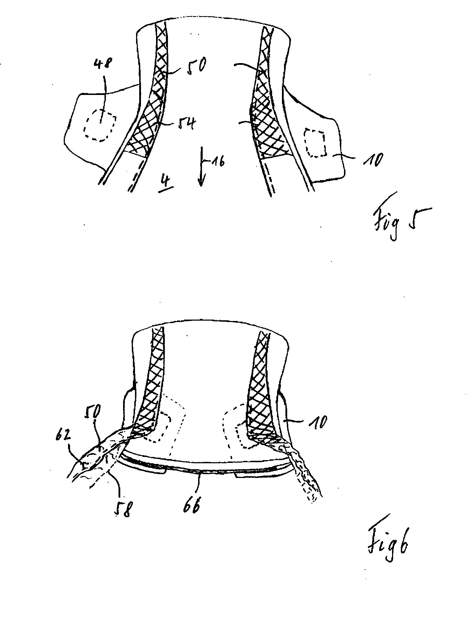

Schließlich zeigen die Figuren 5 und 6 eine schematische perspektivische Betrachtung der Gestalt der Barriereelemente 50, wobei Figur 5 gewissermaßen den Zustand unmittelbar vor Befestigung der Damen- oder Inkontinenzbinde oder -vorlage 2 an einem Unterbekleidungsstück zeigt und Figur 6 den Zustand, bei dem das dargestellte Flügelpaar auf die körperabgewandte Seite der Binde gefaltet und dort an einem schematisch dargestellten Unterbekleidungsstück 66 befestigt ist. In der Darstellung gemäß Figur 5 liegen die Barriereelemente 50 auch in Längsrichtung 16 außerhalb der flächenhaft fixierten Längsabschnitte 24, also in dem Bereich zwischen den Flügeln noch flächenhaft gegen die Oberseite des Bindenhauptteils 4 an. Bei der Darstellung gemäß Figur 6 erkennt man wie die seitlichen Barriereelemente 50 sich in Folge der Spannung ihrer Elastifizierungselemente 62 erheben.

Sollte die Geometrie bzw. der Verlauf des seitlichen Längsrands 24 derart sein, dass die vorderseitige Flanke 32 oder die rückseitige Flanke 40 der Flügel die Gerade oder Tangente 28 schneidet und im weiteren Verlauf die Binde wieder breiter wird in Richtung auf ihr jeweiliges Längsende, so wird dies nicht zur Flügelfläche hinzugerechnet, sondern es handelt sich hierbei vielmehr um ein verbreitertes Ende der Binde ohne Flügelfunktion.

## Patentansprüche

1. Damen- oder Inkontinenzbinde (2) mit um den Steg eines Unterbekleidungsstücks herum faltbaren Flügeln (8, 10), wobei die Binde eine Längsrichtung (16) und eine Querrichtung (18) sowie einen in der Längsrichtung (16) vorderen Endbereich (20) und einen in der Längsrichtung (16) hinteren Endbereich (22) aufweist, mit einem mit seiner längeren Abmessung in der Längsrichtung (16) erstreckten und einen Absorptionskörper (6) für Körperflüssigkeiten aufnehmenden Bindenhauptteil (4) und mit zwei Flügeln (8, 10) auf jeder Längsseite des Bindenhauptteils (4), die ausgehend von dem Bindenhauptteil (4) in Querrichtung (18) vorstehen, so dass sie ein vorderes und ein hinteres Flügelpaar (12, 14) bilden, wobei die Flügel (8, 10) eines Flügelpaars (12, 14) über Haftzonen (48) miteinander oder mit einem Unterbekleidungsstück lösbar verbindbar sind und jeweils von einer vorderseitigen und von einer rückseitigen Flanke (32, 34; 38, 40) begrenzt werden, und mit beidseits entlang von Längsseitenrandbereichen des Bindenhauptteils (4) erstreckten und befestigten Barriereelementen (50), die jeweils an ihrem distalen Längsrand (60) ein gespanntes Elastifizierungsmittel (62) aufweisen, welches bestrebt ist, das betreffende Barriereelement (50) emporzuheben, wobei das jeweilige Barriereelement (50) in einem vorderen und einem hinteren Längsabschnitt (52, 54) in eben gefalteter Konfiguration flächenhaft gegen eine Oberseite des Bindenhauptteils (4) fixiert ist, so dass es sich in diesen Längsabschnitten (52, 54) nicht erheben kann, **dadurch gekennzeichnet, dass** sich der flächenhaft fixierte vordere und hintere Längsabschnitt (52, 54) des jeweiligen Barriereelements (50) ausgehend von dem vorderen bzw. hinteren Endbereich (20, 22) in Längsrichtung (16, P) wenigstens bis zu einem Punkt (64) erstreckt, an dem der benachbarte Flügel (8, 10) die Hälfte seiner Fläche erreicht hat.

2. Damen- oder Inkontinenzbinde nach Anspruch 1, **dadurch gekennzeichnet, dass** sich der flächenhaft fixierte vordere und hintere Längsabschnitt (52, 54) des jeweiligen Barriereelements (50) ausgehend von dem vorderen bzw. hinteren Endbereich (20, 22) in Längsrichtung (16, P) weitestens bis zu einem Punkt erstreckt, an dem der benachbarte Flügel (8, 10) 100%, insbesondere 90%, insbesondere 80% seiner Fläche erreicht hat.

3. Damen- oder Inkontinenzbinde nach Anspruch 1 oder 2, **dadurch gekennzeichnet, dass** das jeweilige Barriereelement (50) im Bereich (56) zwischen den Flügeln (8, 10) nur entlang einer proximalen Linie (58) mit der Oberseite des Bindenhauptteils (4) fixiert ist, so dass es sich mit seinem distalen elastifizierten Längsrand (60) emporheben kann.

4. Damen- oder Inkontinenzbinde nach Anspruch 1, 2 oder 3, **dadurch gekennzeichnet, dass** die Flügel (8) des vorderen Flügelpaars (12) mit wenigstens 90 % ihrer Aufsichtsfläche innerhalb eines Längsabschnittsbereichs (26) der Binde angeordnet sind, der höchstens ein Drittel, insbesondere höchstens ein Viertel der Gesamtlängserstreckung der Damen- oder Inkontinenzbinde erfasst.

5. Damen- oder Inkontinenzbinde nach einem oder mehreren der vorstehenden Ansprüche, **dadurch gekennzeichnet, dass** der vordere Endbereich (20) in Längsrichtung (16) höchstens ein Drittel (1/3), insbesondere höchstens zwei Siebtel (2/7), insbesondere wenigstens ein Siebtel (1/7), insbesondere wenigstens ein Sechstel (1/6), insbesondere wenigstens ein Fünftel (1/5) der Gesamtlängserstreckung der Binde erfasst und die Flügel (8) des vorderen Flügelpaars (12) mit wenigstens 90 % ihrer Aufsichtsfläche in Längsrichtung (16) außerhalb des vorderen Endbereichs (20) angeordnet sind.

6. Damen- oder Inkontinenzbinde nach einem oder mehreren der vorstehenden Ansprüche, **dadurch gekennzeichnet, dass** die Erstreckung der Flügel (8, 10) des vorderen und/oder hinteren Flügelpaars (12, 14) in Querrichtung (18) über die schmalste Stelle (30) der Binde zwischen dem vorderen und dem hinteren Flügelpaar (12, 14) hinaus 20 - 70 mm, insbesondere 20 - 50 mm, insbesondere 25 - 45 mm beträgt.

7. Damen- oder Inkontinenzbinde nach einem oder mehreren der vorstehenden Ansprüche, **dadurch gekennzeichnet, dass** die Aufsichtsfläche jedes Flügels (10) des hinteren Flügelpaars (14) und/oder die Aufsichtsfläche jedes Flügels (8) des vorderen Flügelpaars (12) jeweils einen Anteil von 3-10%, weitere vorzugsweise von 3-8 %, weiter vorzugsweise 3 - 7 %, weiter vorzugsweise von 4 - 6 % bezogen auf die Gesamtfläche der Damen - oder Inkontinenzbinde einnimmt.

8. Damen- oder Inkontinenzbinde nach einem oder mehreren der vorstehenden Ansprüche, **dadurch gekennzeichnet, dass** die Aufsichtsfläche jedes Flügels (8) des vorderen Flügelpaars (12) jeweils 10 - 17 cm², weiter vorzugsweise 11 - 16 cm², weiter vorzugsweise 12 - 15 cm², weiter vorzugsweise 13 - 14 cm² und/oder die jedes Flügels (10) des hinteren Flügelpaars (14) jeweils 10 - 17 cm², weiter vorzugsweise 11 - 16 cm², weiter vorzugsweise 12 - 15 cm², weiter vorzugsweise 13 - 14 cm² beträgt.

9. Damen- oder Inkontinenzbinde nach einem oder mehreren der vorstehenden Ansprüche, **dadurch gekennzeichnet, dass** die vorderseitigen und die rückseitigen Flanken (32, 34; 38, 40) der Flügel (8, 10) des vorderen und/oder hinteren Flügelpaars (12, 14) mit dem überwiegenden Teil ihrer Bahn in einem Winkel (α, β) > 50°, insbesondere > 60°, insbesondere > 65° zur Längsrichtung () verlaufen, wobei der Winkel weiter insbesondere 60 - 85°, insbesondere 60 - 75° beträgt.

10. Damen- oder Inkontinenzbinde nach einem oder mehreren der vorstehenden Ansprüche, **dadurch gekennzeichnet, dass** zwischen der vorderseitigen und der rückseitigen Flanke (32, 34; 38, 40) eines jeweiligen Flügels (8, 10) ein gegenüber diesen kürzerer Flankenabschnitt (42) vorgesehen ist, der insbesondere im Wesentlichen in Längsrichtung (16) verläuft und in den die Flanken (32, 34; 38, 40) verrundet übergehen.

11. Damen- oder Inkontinenzbinde nach einem oder mehreren der vorstehenden Ansprüche, **dadurch gekennzeichnet, dass** ein Krümmungsradius (R) im Übergang zwischen einem im Wesentlichen in der Längsrichtung (16) erstreckten Längsrand (24) des Bindenhauptteils (4) und der vorderseitigen Flanke (32, 38) und/oder zwischen der vorderseitigen Flanke (32, 38) und dem mittleren kürzeren Flankenabschnitt (42) und/oder zwischen dem mittleren kürzeren Flankenabschnitt (42) und der rückseitigen Flanke (34, 40) und/oder zwischen der rückseitigen Flanke (34, 40) und dem im Wesentlichen in der Längsrichtung erstreckten Längsrand (24) des Bindenhauptteils (4) eines jeweiligen Flügels (8, 10) 5 - 12 mm, insbesondere 5 - 10 mm, insbesondere 6 - 9 mm beträgt.

12. Damen- oder Inkontinenzbinde nach einem oder mehreren der vorstehenden Ansprüche, **dadurch gekennzeichnet, dass** die rückseitigen Flanken (34) des vorderen Flügelpaars (12) und die vorderseitigen Flanken (38) des hinteren Flügelpaars (14) mit dem überwiegenden Teil ihrer Bahn gerade verlaufen.

13. Damen- oder Inkontinenzbinde nach einem oder mehreren der vorstehenden Ansprüche, **dadurch gekennzeichnet, dass** die vorderseitigen Flanken (32) des vorderen Flügelpaars (12) und die rückseitigen Flanken (40) des hinteren Flügelpaars (14) bogenförmig gekrümmt verlaufen.

14. Damen- oder Inkontinenzbinde nach einem oder mehreren der vorstehenden Ansprüche, **dadurch gekennzeichnet, dass** die vorderseitigen Flanken (32) des vorderen Flügelpaars (12) und die rückseitigen Flanken (40) des hinteren Flügelpaars (14) einander entsprechend konturiert sind, so dass sie bei Faltung der Binde um eine in Querrichtung verlaufende Achse (44) in Deckung aufeinander bringbar sind.

15. Damen- oder Inkontinenzbinde nach einem oder mehreren der vorstehenden Ansprüche, **dadurch gekennzeichnet, dass** die rückseitigen Flanken (40) des hinteren Flügelpaars (14) in ergonomischer Fortsetzung ihrer Bahn nach innen aufeinander zustreben und so den hinteren Endbereich (22) des Bindenhauptteils (4) begrenzen.

16. Damen- oder Inkontinenzbinde nach einem oder mehreren der vorstehenden Ansprüche, **dadurch gekennzeichnet, dass** die Flügel (8, 10) des vorderen und/oder des hinteren Flügelpaars (12, 14) herstellerseitig auf eine körperzugewandte Oberseite der Binde geschlagen sind und einander dabei insbesondere überlappen und dass eine dann nach oben gewandte Haftbeschichtung bei den jeweiligen Flügeln (8, 10) je Flügelpaar (12, 14) von einem einzigen ablösbaren flächenhaften Schutzelement, insbesondere auf Papier- oder Folienbasis, insbesondere mit Antihaftmitteln beschichtet, überfangen ist, welches beide Flügel (8, 10) erfasst.

## Claims

1. Sanitary or incontinence pad (2) with wings (8, 10) that can be folded about a web of an undergarment, where in the pad has a longitudinal direction (16) and a transverse direction (18) and a front end region (22) in the longitudinal direction (16) and a rear end region (22) in the longitudinal direction (16), with a main pad part (4) which extends with its longer dimension in the longitudinal direction (16) and accommodates an absorbent body (6) for bodily fluids, and with two wings (8,10) on each longitudinal side of the main pad part (4) which starting from the main pad part (4) project in transverse direction (18), so that they form a front and a rear wing pair (12, 14), wherein the wings (8, 10) of a wing pair (12, 14) are detachably connectable to one another or with an undergarment via adhesive zones (48) and are each delimited by a front side flank and by a rear side flank (32,34; 38,40), and with barrier elements (50) which extend and are fastened along longitudinal lateral border regions on both sides of the main pad part (4), which barrier elements (50) have at their distal longitudinal border (60) a tensioned elastifying means (62) which seeks to lift the respective barrier element (50), wherein the respective barrier element (50) is fixed in a front and in a rear longitudinal section (52, 54) against the top side of the main pad part (4) in flatly evenly folded configuration so that it cannot rise up in these longitudinal sections (52, 54), **characterized in that** the extensively fixed front and rear longitudinal section (52, 54) of the respective barrier element (50) starting from the front or rear end region (20, 22) extends in longitudinal direction (16, P) at least up to a point (64) at which the neighboring wing (8,10) has reached half of its surface area.

2. Sanitary or incontinence pad according to claim 1, **characterized in that** the extensively fixed front and rear longitudinal section (52, 54) of the respective barrier element (50) starting from the front or rear end region (20, 22) extends in longitudinal direction (16, P) farthest up to a point at which the neighboring wing (8, 10) has reached 100%, in particular 90%, in particular 80 % of its surface area.

3. Sanitary or incontinence pad according to claim 1 or 2, **characterized in that** the respective barrier element (50) in the region (56) between the wings (8, 10) is only fixed with the top side of the main pad part (4) along a proximal line (58) so that it can rise up with its distal elastified longitudinal border (6).

4. Sanitary or incontinence pad according to claim 1, 2 or 3, **characterized in that** the wings (8) of the front wing pair (12) are arranged with at least 90 % of their top view surface within a longitudinal section region (26) of the pad which is at most one third, in particular at most one quarter of the overall longitudinal extent of the sanitary or incontinence pad.

5. Sanitary or incontinence pad according to one or more of the preceding claims, **characterized in that** the front end region (20) in longitudinal direction (16) occupies at most one third (1/3) in particular at most two seventh (2/7) in particular at least one seventh (1/7) in particular at least one sixth (1/6) in particular at least one fifth (1/5) of the overall longitudinal extent of the pad and the wings (8) of the front wing pair (12) are arranged with at least 90 % of their top view surface in longitudinal direction (16) outside the front end region (20).

6. Sanitary or incontinence pad according to one or more of the preceding claims, **characterized in that** the extent of the wings (8, 10) of the front and/or rear wing pair (122, 14) in transverse direction (18) past the narrowest point (30) of the pad between the front and the rear wing pair (12, 14) is 20-70 mm, in particular 20-50 mm, in particular 25-45 mm.

7. Sanitary or incontinence pad according to one or more of the preceding claims, **characterized in that** the top view surface of each wing (10) of the rear wing pair (14) and/or the top view surface of each wing (8) of the front wing pair (12) each occupies a proportion of 3-10%, further preferably 3-8%, further preferably 3-7%, further preferably 4-6% relative to the overall surface of the sanitary or incontinence pad.

8. Sanitary or incontinence pad according to one or more of the preceding claims, **characterized in that** the top view surface of each wing (8) of the front wing (12) is respectively 10-17 cm², further preferably 11-16 cm², further preferably 12 -15 cm², further preferably 13-14 cm² .

9. Sanitary or incontinence pad according to one or more of the preceding claims, **characterized in that** the front side and the rear side flanks (32, 34; 38, 40) of the wings (8, 10) of the front and/or rear wing (12, 14) extend with the predominant portion of their course in an angle (α, 15) > 50°, in particular > 60°, in particular > 65° to the longitudinal direction (), wherein the angle is further 60-85°, in particular 60 - 75°.

10. Sanitary or incontinence pad according to one or more of the preceding claims, **characterized in that** between the front side and the rear side flank (32, 34; 38, 40) of a respective wing (8, 10) a flank section (42) which is shorter relative to the front and rear side flanks is provided, which extends in particular essentially in longitudinal direction (16) and into which the flanks (32, 34; 38, 40) transition rounded.

11. Sanitary or incontinence pad according to one or more of the preceding claims, **characterized in that** a curve radius (R) in the transition between a longitudinal border (24) of the main pad part, which longitudinal border (24) essentially extends in longitudinal direction (16) and the front side flank (32, 38) and/or between the front side flank (32, 38) and the center shorter flank section (42) and/or between the center shorter flank section (42) and the rear side flank (34, 40) and/or between the rear side flank (34, 40) and the longitudinal border (24) of the main pad part (4) which longitudinal border (24) extends essentially in the longitudinal direction of a respective wing (8,10) is 5-12 mm, in particular 5-10 mm, in particular 6-9 mm.

12. Sanitary or incontinence pad according to one or more of the preceding claims, **characterized in that** the rear side flanks (34) of the front wing pair (12) and the front side flanks (38) of the rear wing pair (14) extend straight with the predominant portion of their course.

13. Sanitary or incontinence pad according to one or more of the preceding claims, **characterized in that** the front side flanks (32) of the front wing pair (12) and the rear side flanks (40) of the rear wing pair (14) extend curved arch shaped.

14. Sanitary or incontinence pad according to one or more of the preceding claims, **characterized in that** the front side flanks (32) of the front wing pair (12) and the rear side flanks (40) of the rear wing pair (14) are contoured to correspond to one another, so that they can be brought to align with each other when folding the pad about an axis (44) which extends in transverse direction.

15. Sanitary or incontinence pad according to one or more of the preceding claims, **characterized in that** the rear side flanks (40) of the rear wing pair (14) extend inwardly toward one another in ergonomic extension of their course and in this way delimit the rear end region (22) of the main pad part (4).

16. Sanitary or incontinence pad according to one or more of the preceding claims, **characterized in that** the wings (8, 10) of the front and/or the rear wing pair (12, 14) are folded by the manufacturer onto a body facing top side of the pad and in particular overlap one another and **in that** an adhesive coating which then faces upwards is covered in the respective wings (8, 10) per wing pair (12, 14) by a single detachable two dimensional protective element, in particular on paper or foil basis, in particular coated with anti adhesive means, which protective element covers both wings (8, 10).

## Revendications

1. Serviette hygiénique ou de protection contre l'incontinence (2), ayant des ailettes (8, 10) aptes à être repliées autour de l'entrejambe d'un sous-vêtement, ladite serviette présentant une direction longitudinale (16) et une direction transversale (18) ainsi qu'une zone terminale (20) avant dans ladite direction longitudinale (16) et une zone terminale (22) arrière dans ladite direction longitudinale (16), avec une partie principale de serviette (4) qui, avec sa dimension plus longue, s'étend dans la direction longitudinale (16) et qui reçoit un corps d'absorption (6) pour fluides corporels, et avec deux ailettes (8, 10) de chaque grand côté de ladite partie principale de serviette (4), qui font saillie dans la direction transversale (18) à partir de la partie principale de serviette (4) de sorte qu'elles forment des paires d'ailettes avant et arrière (12, 14), lesdites ailettes (8, 10) d'une paire d'ailettes (12, 14) pouvant être reliées, par des zones adhésives (48), de manière détachable entre elles ou à un sous-vêtement et étant délimitées chacune d'un flanc frontal et d'un flanc arrière (32, 34; 38, 40), ainsi qu'avec des éléments barrière (50) qui s'étendent et sont fixés de part et d'autre le long de zones marginales de grand côté de ladite partie principale de serviette (4) et qui présentent chacun, sur leur bord longitudinal distal (60), un moyen d'élastification (62) tendu qui tend à soulever l'élément barrière (50) respectif, l'élément barrière (50) respectif étant fixé, dans des portions longitudinales avant et arrière (52, 54), en configuration pliée de manière plane, en nappe contre une face supérieure de ladite partie principale de serviette (4) de sorte qu'il ne puisse pas se lever dans ces portions longitudinales (52, 54), **caractérisée par le fait que** lesdites portions longitudinales avant et arrière (52, 54) de l'élément barrière (50) respectif, qui sont fixées en nappe, s'étendent à partir de ladite zone terminale avant ou bien arrière (20, 22), dans la direction longitudinale (16, P), au moins jusqu'à un point (64) sur lequel l'ailette (8, 10) voisine a atteint la moitié de sa surface.

2. Serviette hygiénique ou de protection contre l'incontinence selon la revendication 1, **caractérisée par le fait que** lesdites portions longitudinales avant et arrière (52, 54) de l'élément barrière (50) respectif, qui sont fixées en nappe, s'étendent à partir de ladite zone terminale avant ou bien arrière (20, 22), dans la direction longitudinale (16, P), tout au plus jusqu'à un point sur lequel l'ailette (8, 10) voisine a atteint 100 %, en particulier 90 %, en particulier 80 % de sa surface.

3. Serviette hygiénique ou de protection contre l'incontinence selon la revendication 1 ou 2, **caractérisée par le fait que**, dans la zone (56) située entre les ailettes (8, 10), l'élément barrière (50) respectif n'est fixé que le long d'une ligne proximale (58) avec la face supérieure de la partie principale de serviette (4) de sorte qu'il puisse se lever par son bord longitudinal (60) distal élastifié.

4. Serviette hygiénique ou de protection contre l'incontinence selon la revendication 1, 2 ou 3, **caractérisée par le fait que** les ailettes (8) de la paire d'ailettes avant (12) sont disposées, avec au moins 90 % de leur surface de vue en plan, à l'intérieur d'une zone de portion longitudinale (26) de la serviette, qui comprend tout au plus un tiers, en particulier tout au plus un quart de l'extension longitudinale totale de la serviette hygiénique ou de protection contre l'incontinence.

5. Serviette hygiénique ou de protection contre l'incontinence selon l'une ou plusieurs des revendications précédentes, **caractérisée par le fait que** la zone terminale avant (20) comprend, dans la direction longitudinale (16), tout au plus un tiers (1/3), en particulier tout au plus deux septièmes (2/7), en particulier au moins un septième (1/7), en particulier au moins un sixième (1/6), en particulier au moins un cinquième (1/5) de l'extension longitudinale totale de la serviette et que les ailettes (8) de la paire d'ailettes avant (12) sont disposées, avec au moins 90 % de leur surface de vue en plan, dans la direction longitudinale (16), à l'extérieur de la zone terminale avant (20).

6. Serviette hygiénique ou de protection contre l'incontinence selon l'une ou plusieurs des revendications précédentes, **caractérisée par le fait que** l'extension des ailettes (8, 10) des paires d'ailettes avant et/ou arrière (12, 14) dans la direction transversale (18), au-delà de l'endroit (30) le plus étroit de la serviette entre les paires d'ailettes avant et arrière (12, 14), est comprise entre 20 et 70 mm, en particulier entre 20 et 50 mm, en particulier entre 25 et 45 mm.

7. Serviette hygiénique ou de protection contre l'incontinence selon l'une ou plusieurs des revendications précédentes, **caractérisée par le fait que** la surface de vue en plan de chaque ailette (10) de la paire d'ailette arrière (14) et/ou la surface de vue en plan de chaque ailette (8) de la paire d'ailettes avant (12) occupe chacune une part comprise entre 3 et 10 %, de préférence encore entre 3 et 8 %, de préférence encore entre 3 et 7 %, de préférence encore entre 4 et 6 %, par rapport à la surface totale de la serviette hygiénique ou de protection contre l'incontinence.

8. Serviette hygiénique ou de protection contre l'incontinence selon l'une ou plusieurs des revendications précédentes, **caractérisée par le fait que** la surface de vue en plan de chaque ailette (8) de la paire d'ailettes avant (12) fait chacune entre 10 et 17 cm², de préférence encore entre 11 et 16 cm², de préférence encore entre 12 et 15 cm², de préférence encore entre 13 et 14 cm ², et/ou celle de chaque ailette (10) de la paire d'ailettes arrière (14) fait chacune entre 10 et 17 cm², de préférence encore entre 11 et 16 cm², de préférence encore entre 12 et 15 cm², de préférence encore entre 13 et 14 _{cm}².

9. Serviette hygiénique ou de protection contre l'incontinence selon l'une ou plusieurs des revendications précédentes, **caractérisée par le fait que** la majeure partie de la voie des flancs frontaux et des flancs arrière (32, 34; 38, 40) des ailettes (8, 10) des paires d'ailettes avant et/ou arrière (12, 14) s'étend à un angle (α, β) > 50°, en particulier > 60°, en particulier > 65° par rapport à la direction longitudinale (16), ledit angle étant, en particulier encore, compris entre 60 et 85°, en particulier entre 60 et 75°.

10. Serviette hygiénique ou de protection contre l'incontinence selon l'une ou plusieurs des revendications précédentes, **caractérisée par le fait qu'**entre le flanc frontal et le flanc arrière (32, 34; 38, 40) d'une ailette (8, 10) respective est prévue une portion de flanc (42) qui est plus courte par rapport à ceux-ci et qui s'étend, en particulier, pour l'essentiel dans la direction longitudinale (16) et auquel lesdits flancs (32, 34; 38, 40) sont contigus tout en étant arrondis.

11. Serviette hygiénique ou de protection contre l'incontinence selon l'une ou plusieurs des revendications précédentes, **caractérisée par le fait qu'**un rayon de courbure (R) dans la transition entre un bord longitudinal (24) de la partie principale de serviette (4), qui s'étend pour l'essentiel dans la direction longitudinale (16), et le flanc frontal (32, 38) et/ou entre le flanc frontal (32, 38) et ladite portion de flanc (42) centrale plus courte et/ou entre ladite portion de flanc (42) centrale plus courte et le flanc arrière (34, 40) et/ou entre le flanc arrière (34, 40) et le bord longitudinal (24) de la partie principale de serviette (4) d'une ailette (8, 10) respective, qui s'étend pour l'essentiel dans la direction longitudinale, est compris entre 5 et 12 mm, en particulier entre 5 et 10 mm, en particulier entre 6 et 9 mm.

12. Serviette hygiénique ou de protection contre l'incontinence selon l'une ou plusieurs des revendications précédentes, **caractérisée par le fait que** la majeure partie de la voie des flancs arrière (34) de la paire d'ailettes avant (12) ainsi que des flancs frontaux (38) de la paire d'ailette arrière (14) s'étend de manière droite.

13. Serviette hygiénique ou de protection contre l'incontinence selon l'une ou plusieurs des revendications précédentes, **caractérisée par le fait que** les flancs frontaux (32) de la paire d'ailettes avant (12) et les flancs arrière (40) de la paire d'ailette arrière (14) s'étendent de manière courbée en arc.

14. Serviette hygiénique ou de protection contre l'incontinence selon l'une ou plusieurs des revendications précédentes, **caractérisée par le fait que** les flancs frontaux (32) de la paire d'ailettes avant (12) et les flancs arrière (40) de la paire d'ailette arrière (14) présentent des contours qui correspondent entre eux de sorte que, lorsque la serviette est pliée sur un axe (44) s'étendant dans la direction transversale, ils puissent être mis en coïncidence l'un sur l'autre.

15. Serviette hygiénique ou de protection contre l'incontinence selon l'une ou plusieurs des revendications précédentes, **caractérisée par le fait que** les flancs arrière (40) de la paire d'ailettes arrière (14), en prolongement ergonomique de leur voie, s'étendent vers l'intérieur l'un en direction de l'autre et délimitent ainsi la zone terminale arrière (22) de la partie principale de serviette (4).

16. Serviette hygiénique ou de protection contre l'incontinence selon l'une ou plusieurs des revendications précédentes, **caractérisée par le fait que** les ailettes (8, 10) des paires d'ailettes avant et/ou arrière (12, 14) sont rabattues, de la part du fabricant, sur une face supérieure de la serviette qui montre vers le corps et, se faisant, en particulier se chevauchent les unes les autres, et qu'un revêtement adhésif montrant alors vers le haut, dans les ailettes (8, 10) respectives par paire d'ailettes (12, 14), est recouvert d'un seul élément protecteur détachable en nappe, en particulier à base de papier ou de feuille, en particulier revêtu d'agents antiadhésifs, qui s'étend sur les deux ailettes (8, 10).
